# EUROPÄISCHE PATENTSCHRIFT

(11) **EP 0 568 819 B1**
(45) Veröffentlichungstag und Bekanntmachung des Hinweises auf die Patenterteilung: **03.01.1996**
(21) Anmeldenummer: 93105656.8
(22) Anmeldetag: 06.04.1993
(51) Int. Cl.: C07C 45/71, C07C 47/27, C07C 39/11

(54) **Verfahren zur Herstellung von 3-(Hydroxyphenyl)-propionaldehyden und gegebenenfalls der Herstellung von 3-(Hydroxyphenyl)-propanolen**
Process for the preparation of 3-(hydroxyphenyl)-propionaldehydes and optionally the preparation of 3-(hydroxyphenyl)-propanols
Procédé pour la préparation d'aldéhydes 3-(hydroxyphényl) et éventuellement la préparation de propanols 3-(hydroxyphényl)

(30) Priorität: 25.04.1992 DE 4213750
(43) Veröffentlichungstag der Anmeldung: 10.11.1993
(73) Patentinhaber: BASF Aktiengesellschaft, D-67063 Ludwigshafen (DE)
(72) Erfinder: Merger, Franz, Dr., W-6710 Frankenthal (DE); Schmidt-Radde, Martin, Dr., W-6711 Beindersheim (DE)

(56) Entgegenhaltungen:
- EP-A- 0 027 426
- GB-A- 2 026 479

## Beschreibung

Die vorliegende Erfindung betrifft ein Verfahren zur Herstellung von 3-(Hydroxyphenyl)-propionaldehyden und gegebenenfalls der Herstellung von 3-(Hydroxyphenyl)-propanolen durch Umsetzung von Phenolen in Gegenwart eines basischen Katalysators bei erhöhten Temperaturen und gegebenenfalls der katalytischen Hydrierung der entstandenen 3-(Hydroxyphenyl)-propionaldehyde in Gegenwart eines Hydrierkatalysators.

Aus der US-A-4 091 225 ist die Herstellung von 3-(Hydroxyphenyl)-propionaldehyden I durch die Umsetzung von 3,5-Di-t-Butyl-4-hydroxybenzylchlorid mit 2,2-Dialkylalkanalen unter Phasentransferbedingungen bekannt.

Aus der GB-A-1 455 766 ist die Herstellung von 3-(Hydroxyphenyl)propionaldehyden I durch die alkalihydroxid-katalysiert Umsetzung von 3,5-Dialkyl-4-hydroxybenzyl-N,N-dialkyldithiocarbamate mit 2,2-Dialkylalkanalen bekannt.

Aus der EP-A-27 426 schließlich ist die Bildung von 3-(Hydroxyphenyl)propionaldehyden I aus 3,5-Dialkyl-4-hydroxybenzyl-alkylethern und 2,2-Dialkylalkanalen unter der katalytischen Wirkung von Alkalihydroxiden bzw. -alkoholaten bekannt.

Allen genannten Verfahren weisen den Nachteil auf, daß sie von funktionalisierten Dialkylphenol-Derivaten ausgehen, die erst aufwendig aus Dialkylphenolen hergestellt werden müssen.

Ein weiterer Nachteil besteht darin, daß die Katalysatoren neutralisiert werden müssen, d.h. nicht rückführbar sind.

Für die Umwandlung der 3-(Hydroxyphenyl)-propionaldehyde I in die 3-(Hydroxyphenyl)-propanole II durch katalytische Hydrierung müssen nach US-A-4 091 225 die isolierten und gereinigten 3-(Hydroxyphenyl)propionaldehyde I eingesetzt werden.

Der vorliegenden Erfindung lag die Aufgabe zugrunde, den zuvor genannten Nachteilen abzuhelfen.

Demgemäß wurde ein neues und verbessertes Verfahren zur Herstellung von 3-(Hydroxyphenyl)-propionaldehyden allgemeinen Formel I 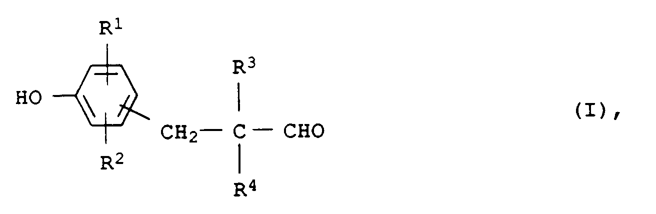
und gegebenenfalls Herstellung von 3-(Hydroxyphenyl)-propanolen der allgemeinen Formel II 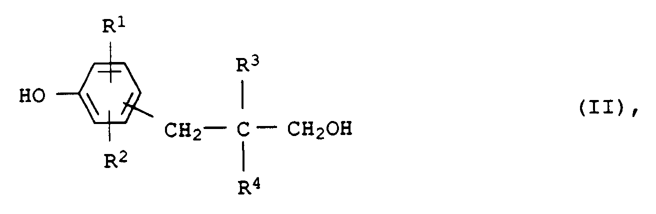
in der
- R¹,R²,R³,R⁴: Wasserstoff, C₁- bis C₂₀-Alkyl, C₃- bis C₂₀-Cycloalkyl, C₄- bis C₂₀-Alkyl-cycloalkyl, C₄- bis C₂₀-Cycloalkyl-alkyl oder C₅- bis C₂₀-Alkyl- cycloalkyl-alkyl,
- R³,R⁴: Aryl, C₇- bis C₂₀-Aralkyl, Heterocycloalkyl oder C₃- bis C₂₀-Heterocycloalkyl-alkyl, bedeuten, gefunden, welches dadurch gekennzeichnet ist, daß man

a) Phenole der allgemeinen Formel III 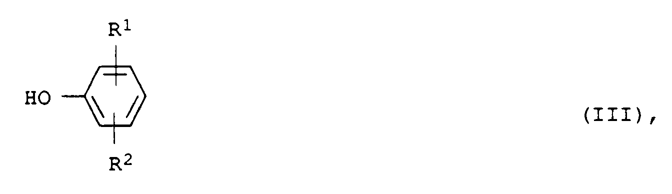 in der
   R¹ und R² die oben genannten Bedeutungen haben, mit 3- Hydroxy-propionaldehyden der allgemeinen Formel IV 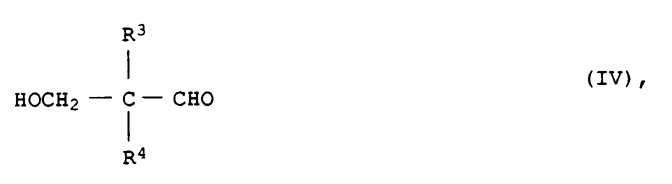 in der R³ und R⁴ die oben genannten Bedeutungen haben, in Gegenwart eines basischen Katalysators bei Temperaturen von 90 bis 230°C und Drücken von 0,01 bis 50 bar umsetzt, und gegebenenfalls
b) die entstandenen 3-(Hydroxyphenyl)-propionaldehyde der allgemeinen Formel I 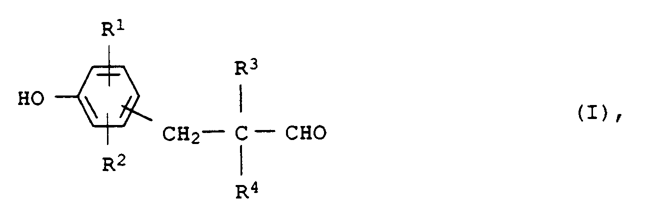 in der R¹, R², R³ und R⁴ die oben genannten Bedeutungen haben, in Gegenwart eines Hydrierkatalysators mit Wasserstoff bei Temperaturen von 0 bis 250°C und Drücken von 0,1 bis 300 bar behandelt.

Das erfindungsgemäße Verfahren kann wie folgt durchgeführt werden:
a) Die Phenole III kann man mit 3-Hydroxy-propionaldehyden IV in Gegenwart eines basischen Katalysators und vorzugsweise unter Zusatz eines inerten Lösungsmittels bei Temperaturen von 90 bis 230°C, bevorzugt 110 bis 210°C, besonders bevorzugt 140 bis 190°C und Drücken von 0,01 bis 50 bar, bevorzugt 0,1 bis 5 bar, besonders bevorzugt unter Eigendruck des Systems umsetzen.
   Die Reaktionsdauer hängt vom Substitutionsmuster des eingesetzten Dialkylphenols ab und beträgt in der aller Regel zwischen 0,2 und 200 h.
   Die Umsetzung kann kontinuierlich oder diskontinuierlich, vorzugsweise diskontinuierlich durchgeführt werden, wobei alle üblichen Reaktoren verwendet werden können.
   Das Molverhältnis von Phenol III zum 3-Hydroxy-propionaldehyd IV liegt in der Regel im Bereich von 0,5 : 1 bis 1,5 : 1, vorzugsweise 0,8 : 1 bis 1,2 : 1.
   Als basische Katalysatoren eignen sich beispielsweise Amine, vorzugsweise sekundäre Amine. Als sekundäre Amine kommen heterocyclische, aliphatische und cycloaliphatische Amine mit 2 bis 20 Kohlenstoffatomen, bevorzugt 4 bis 15 Kohlenstoffatomen, in Betracht, wobei aliphatische sekundäre bevorzugt eingesetzt werden. Als Beispiele seien folgende sekundäre Amine genannt: Dimethylamin, Diethylamin, Di-n-Propylamin, Di-Isopropylamin, Di-n-Butylamin, Di-Isobutylamin, Methyl-ethylamin, Methyl-n-propylamin, Methyl-isopropylamin, Methyl-n-butylamin, Methyl-isobutylamin, Ethyl-n-propylamin, Ethyl-isopropylamin, Ethyl-n-butylamin, Methylcyclohexylamin, Pyrrolidin, Piperidin, Piperazin, Morpholin, N-Methyl-ethanolamin und Diethanolamin.
   Das Molverhältnis von basischem Katalysator zum 3-Hydroxy-propionaldehyd IV liegt in der Regel im Bereich von 0,01 : 1 bis 0,5 : 1, bevorzugt 0,05 : 1 bis 0,25 : 1. Höhere Katalysatormengen sind möglich, bringen jedoch in der Regel keine Vorteile.
   Die 3-Hydroxy-propionaldehyde IV können, wie z.B. in DE-A-17 93 512 und DE-A-19 57 301 beschrieben, aus Aldehyden mit einem Beta-Wasserstoffatom und wäßrigem Formaldehyd unter Trialkylaminkatalyse hergestellt werden. Dabei ist sowohl das Rohprodukt, als auch das gereinigte Produkt der Umsetzung des jeweiligen Isoaldehyds mit Formaldehyd geeignet. Üblicherweise werden 3-Hydroxy-propionaldehyde IV mit einem Wassergehalt von 0 bis 50 Gew.-%, vorzugsweise 4 bis 20 Gew.-% verwendet.
   Als inerte Lösungsmittel eignen sich inerte organische Lösungsmittel, wobei vorzugsweise solche Lösungsmittel verwendet werden, die mit Wasser gut mischbar sind. Als Beispiele seien genannt: (Cyclische) Ether wie Tetrahydrofuran und Dioxan, sowie C₁- bis C₃₀-Alkanole wie Methanol, Ethanol, Propanol, Isopropanol, Ethylenglykol, Propylenglykol, Ethylenglykol-mono-ethylether, Methylglykol. Besonders bevorzugt sind C₁- bis C₃-Alkanole.
   Die Lösungsmittelmenge sollte so bemessen sein, daß der Lösungsmittelgehalt des Reaktionsgemisches 5 bis 95 Gew.-%, vorzugsweise 40 bis 90 Gew.-% beträgt.
   Aus dem Reaktionsgemisch können die 3-(Hydroxyphenyl)-propionaldehyde I auf einfache Weise nach an sich herkömmlichen Methoden isoliert, beispielsweise durch Destillation und/oder Kristallisation.
b) Gegebenenfalls können die entstandenen 3-(Hydroxyphenyl)-propionaldehyde I in Gegenwart eines Hydrierkatalysators, bevorzugt eines heterogenen Katalysator mit Wasserstoff bei Temperaturen von 0 bis 250°C, bevorzugt 20 bis 200°C und Drücken von 0,1 bis 300 bar, bevorzugt 1 bis 200 bar behandeln. Dabei kann der Hydrierkatalysator in der Reaktionsmischung suspendiert oder in einem Festbett angeordnet werden.

Für die Hydrierung der 3-(Hydroxyphenyl)-propionaldehyde I zu den 3-(Hydroxyphenyl)-propanolen II können prinzipiell alle üblichen Hydrierkatalysatoren verwendet werden, beispielsweise Nickel, Kobalt, Kupfer, Mangan, Molybdän Rhenium und/oder die Platinmetalle Palladium, Platin und Ruthenium enthaltende Katalysatoren. Dabei können sowohl die reinen Metalle, feinverteilt oder in Form von Netzen oder anderen Gebilden hoher Oberfläche, eingesetzt werden, als auch Katalysatoren die mehrere dieser Metalle enthalten. Die Hydrierkatalysatoren können als Vollkatalysatoren oder in Form von Trägerkatalysatoren eingesetzt werden. Es können an sich übliche Trägermaterialien für diese Trägerkatalysatoren verwendet werden, wie Siliciumdioxid, Aluminiumoxide, Zirkondioxid, Titandioxide, Aktivkohle, Bariumsulfat, Bariumcarbonat, Calciumcarbonat und ähnliche. Vorzugsweise werden im erfindungsgemäßen Verfahren Nickel- und/oder Kupfer- und/oder Kobalt-haltige und/oder Rutheniumkatalysatoren eingesetzt. Besonders bevorzugt sind Nickel- und Rutheniumkatalysatoren, bespielsweise Raney-Nickel und Ruthenium/Aktivkohle.

Aus dem Hydriergemisch kann das 3-(Hydroxyphenyl)-propanol II nach an sich herkömmlichen Methoden isoliert werden, beispielsweise durch Destillation und/oder Kristallisation.

Die Substituenten R¹, R², R³ und R⁴ in den Verbindungen der Formeln I und II haben folgende Bedeutungen:
- R¹,R²,R³,R⁴: - unabhängig voneiander
- Wasserstoff,
- C₁- bis C₂₀-Alkyl, bevorzugt C₁- bis C₁₂-Alkyl wie Methyl, Ethyl, n-Propyl, iso-Propyl, n-Butyl, iso-Butyl, sec. -Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- C₄- bis C₂₀-Alkyl-cycloalkyl, bevorzugt 2, 6-Dimethylcyclohexylmethyl,
- C₄- bis C₂₀-Cycloalkyl-alkyl, bevorzugt Cyclopropylmethyl, Cyclobutylmethyl, Cyclopentylmethyl und Cyclohexylmethyl,
- C₅- bis C₂₀-Alkyl-cycloalkyl-alkyl,
- R³,R⁴: - Aryl wie Phenyl, 1-Naphthyl, 2-Naphthyl, 9-Naphthyl und Biphenyl, bevorzugt Phenyl,
- C₇- bis C₂₀-Aralkyl, bevorzugt C₇- bis C₁₂-Aralkyl wie Benzyl, Phenyl-ethyl, 1-Naphthyl-methyl und Biphenyl-methyl;
- Heterocycloalkyl, wie ein 5- oder 6-gliedriger Ring mit einem oder zwei O-, N- und/oder S-Atomen im Ring, der aromatisch oder nicht aromatisch sein kann, wie 2- oder 3-Furyl, 2- oder 3-Thienyl, 2- oder 3-Pyrrolyl, 2- oder 4-Imidazolyl, 2- oder 3-Oxazolyl, 2- oder 3-Thiazolyl, Pyridinyl, Morpholyl, Thiomorpholyl und Pyrazolyl,
- C₃- bis C₂₀-Heterocycloalkyl-alkyl.

Als Phenole III eignen sich vorzugsweise solche, die 2,6-oder 2,4- dialkylsubstituiert sind, wobei R¹ und R² unabhängig voneinander jeweils einen Alkylrest mit 1 bis 6 Kohlenstoffatomen oder einen cycloaliphatischen Rest mit 4 bis 12 Kohlenstoffatomen bedeuten.

Geeignete Dialkylphenole III sind z.B. 2,6-Dimethyl-, 2,6-Diethyl-, 2,6-Di-n-propyl-, 2,6-Diisopropyl-, 2,6-Di-n-butyl-, 2,6-Di-isobutyl-, 2,6-Di-t-butyl, 2,4-Dimethyl-, 2,4-Diethyl-, 2,4-Di-n-propyl-, 2,4- Diisopropyl-, 2,4-Di-n-butyl-, 2,4-Di-isobutyl-, 2,4-Di-t-butyl, 2-Methyl-6-ethyl, 2-Methyl-6-isopropyl, 2-Methyl-6-t-butyl, 2-Methyl-4-ethyl, 2-Methyl-4-isopropyl, 2-Methyl-4-t-butyl-phenol.

Bevorzugte 3-Hydroxy-propionaldehyde IV sind Beta-Hydroxyaldehyde z.B. 2,2-Dimethyl-3-hydroxypropanal, 2-Ethyl-2-methyl-3-hydroxypropanal, 2-Methyl-2-propyl-3-hydroxypropanal, 2-Butyl-2-methyl-3-hydroxy- propanal, 2-Butyl-2-ethyl-3-hydroxypropanal, 2-Ethyl-2-propyl- 3- hydroxypropanal, 2-Ethyl-2-hexyl-3-hydroxypropanal, 2-Ethyl-2-isopropyl-3-hydroxypropanal, 2-Methyl-2-phenyl-3-hydroxypropanal, 2-Methyl-2-(alpha-Naphtyl)-3-hydroxypropanal, 2-Cyclohexyl- 2- methyl-3-hydroxypropanal, 2-Ethyl-2-cyclohexyl-3-hydroxypropanal, 2-Hydroxymethyl-cyclopentyl-, 2-Hydroxymethyl-cyclohexyl-, 2-Hydroxymethyl-cyclooctyl-, 2-Hydroxymethyl-cyclododecylaldehyd, 2-Methyl-2-pyridl-3-hydroxypropanal, 2-Furyl-2-methyl-3-hydroxypropanal.

Die 3-(Hydroxyphenyl)-propionaldehyde I sind vielseitig verwendbare Zwischenprodukte (Aminophenole, Carbonate, Polymerkomponenten).

Die 3-(Hydroxyphenyl)-propanole II sind als einpolymrisierbare Antioxidantien verwendbar.

### Beispiele

### Beispiel 1

125 g (1 mol) 80-%-iger wäßriger 2,2-Dimethyl-3-hydroxypropanal (rohes Synthesegemisch auf 20 % Wassergehalt andestilliert), 109 g (0,9 mol) 2,6-Dimethylphenol, 12 g (0,11 mol) 40 %-iges wäßriges Dimethylamin und 700 g Methanol werden in einem Rührautoklaven bei 180°C und 20 bis 30 bar Eigendruck 10 h umgesetzt. Die destillative Aufarbeitung des Reaktionsgemisches ergibt 168 g (91 %) 3-(3,5-Dimethyl-4-hydroxyphenyl)-2,2-dimethylpropanal (Sdp.: 140 bis 142°C; Smp.: 72 bis 73°C).

### Beispiel 2

125 g (1 mol) 80-%-iger wäßriger 2,2-Dimethyl-3-hydroxypropanal (rohes Synthesegemisch auf 20 % Wassergehalt andestilliert), 185 g (0,9 mol) 2,6-Di-t-butylphenol, 12 g (0,11 mol) 40 %-iges wäßriges Dimethylamin und 700 g Methanol werden in einem Rührautoklaven bei 180°C und 20 bis 30 bar Eigendruck 10 h umgesetzt. Die destillative Aufarbeitung des Reaktionsgemisches ergibt 238 g (92 %) 3-(3,5-Di-t-butyl-4-hydroxy- phenyl)-2,2-dimethylpropanal (Sdp.: 126 bis 127°C; Smp. : 75 bis 77°C).

### Beispiel 3

125 g (1 mol) 80-%-iger wäßriger 2,2-Dimethyl-3-hydroxypropanal (rohes Synthesegemisch auf 20 % Wassergehalt andestilliert), 185 g (0,9 mol) 2,4-Di-t-butylphenol, 24 g (0,22 mol) 40 %-iges wäßriges Dimethylamin und 700 g Methanol werden in einem Rührautoklaven bei 20 bis 30 bar Eigendruck 20 h bei 160°C und nochmals 20 h bei 180°C umgesetzt. Die destillative Aufarbeitung des Reaktionsgemisches ergibt 159 g (61 %) 3-(3,5-Di-t-butyl-2-hydroxyphenyl)-2,2-dimethylpropanal (Sdp.: 115 bis 116°C).

### Beispiel 4

125 g (1 mol) 80-%-iger wäßriger 2,2-Dimethyl-3-hydroxypropanal (rohes Synthesegemisch auf 20 % Wassergehalt andestilliert), 109 g (0,9 mol) 2,6-Dimethylphenol, 12 g (0,11 mol) 40 %-iges wäßriges Dimethylamin und 700 g Methanol werden in einem Rührautoklaven bei 180°C und 20 bis 30 bar Eigendruck 10 h umgesetzt. Nach Abkühlen und Entspannen des Autoklaven setzt man dem Reaktionsgemisch 19 g Ra-Ni (0,02 Teile bez. auf Gesamtansatz) zu und hydriert 5 h bei 80°C und 80 bar Wasserstoffdruck. Die destillative Aufarbeitung des Hydrieraustrages ergibt 174 g (92 %) 3-(3,5-Dimethyl-4-hydroxyphenyl)- 2,2-dimethylpropanol (Sdp.: 172 bis 174°C; Smp.: 133 bis 134°C).

### Beispiel 5

125 g (1 mol) 80-%-iger wäßriger 2,2-Dimethyl-3-hydroxypropanal (rohes Synthesegemisch auf 20 % Wassergehalt andestilliert), 185 g (0,9 mol) 2,6-Di-t-butylphenol, 12 g (0,11 mol) 40 %-iges wäßriges Dimethylamin und 700 g Methanol werden in einem Rührautoklaven bei 180°C und 20 bis 30 bar Eigendruck 10 h umgesetzt. Nach Abkühlen und Entspannen des Autoklaven setzt man dem Reaktionsgemisch 28 g Ra-Ni (0,03 Teile bez. auf Gesamtansatz) zu und hydriert 5 h bei 80°C und 80 bar Wasserstoffdruck. Die destillative Aufarbeitung des Hydrieraustrages ergibt 239 g (91 %) 3-(3,5-Di-t-butyl-4-hydroxyphenyl)-2,2-dimethylpropanol (Sdp.: 142 bis 145°C; Smp.: 84 bis 86°C).

### Beispiel 6

125 g (1 mol) 80-%-iger wäßriger 2,2-Dimethyl-3-hydroxypropanal (rohes Synthesegemisch auf 20 % Wassergehalt andestilliert), 109 g (0,9 mol) 2,4-Dimethylphenol, 24 g (0,22 mol) 40 %-iges wäßriges Dimethylamin und 700 g Methanol werden in einem Rührautoklaven bei 20 bis 30 bar Eigendruck 20 h bei 160°C und nochmals 20 h bei 180°C umgesetzt. Nach Abkühlen und Entspannen des Autoklaven setzt man dem Reaktionsgemisch 95 g Ra-Ni (0,1 Teile bez. auf Gesamtansatz) zu und hydriert 20 h bei 140°C und 80 bar Wasserstoffdruck. Nach Filtration und destillativer Abtrennung von flüchtigen Bestandteilen wird der Rückstand aus Methanol/Wasser 10 : 3 umkristallisiert. Man erhält 112 g (60 %) 3-(3,5-Di- methyl-2-hydroxyphenyl)-2,2-dimethylpropanol (Smp.: 117°C).

## Patentansprüche

1. Verfahren zur Herstellung von 3-(Hydroxyphenyl)-propionaldehyden allgemeinen Formel I 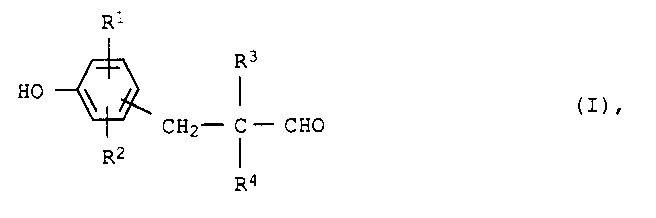 und gegebenenfalls Herstellung von 3-(Hydroxyphenyl)-propanolen der allgemeinen Formel II 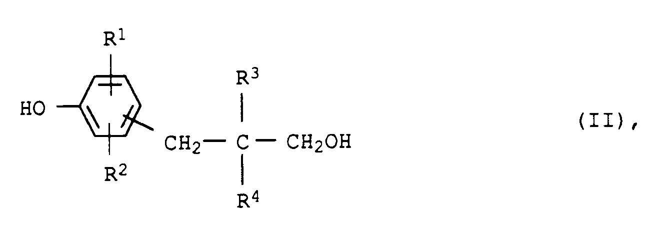 in der
R¹,R²,R³,R⁴ Wasserstoff, C₁- bis C₂₀-Alkyl, C₃- bis C₂₀-Cycloalkyl, C₄- bis C₂₀-Alkyl-cycloalkyl, C₄- bis C₂₀-Cycloalkyl-alkyl oder C₅- bis C₂₀-Alkylcycloalkyl-alkyl,
R³,R⁴ Aryl, C₇- bis C₂₀-Aralkyl, Heterocycloalkyl oder C₃- bis C₂₀-Heterocycloalkyl-alkyl,
bedeuten, dadurch gekennzeichnet, daß man
a) Phenole der allgemeinen Formel III 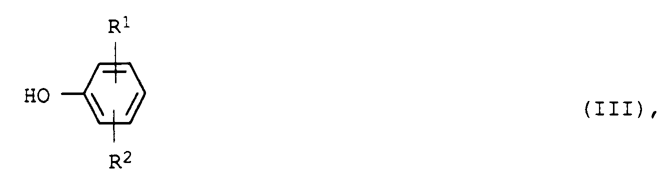 in der R¹ und R² die oben genannten Bedeutungen haben, mit 3-Hydroxy-propionaldehyden der allgemeinen Formel IV 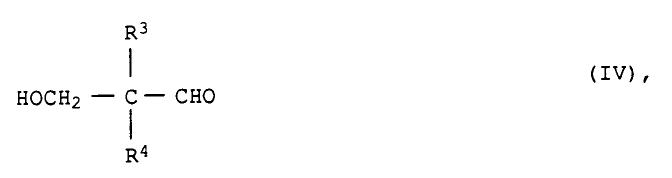 in der R³ und R⁴ die oben genannten Bedeutungen haben, in Gegenwart eines basischen Katalysators bei Temperaturen von 90 bis 230°C und Drücken von 0,01 bis 50 bar umsetzt, und gegebenenfalls
b) die entstandenen 3-(Hydroxyphenyl)-propionaldehyde der allgemeinen Formel I 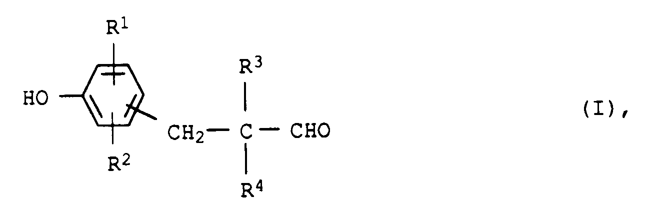 in der R¹, R², R³ und R⁴ die oben genannten Bedeutungen haben, in Gegenwart eines Hydrierkatalysators mit Wasserstoff bei Temperaturen von 0 bis 250°C und Drücken von 0,1 bis 300 bar behandelt.

2. Verfahren zur Herstellung von 3-(Hydroxyphenyl)-propionaldehyden I nach Anspruch 1, dadurch gekennzeichnet, daß man die Umsetzung bei Temperaturen von 110 bis 210°C durchführt.

3. Verfahren zur Herstellung von 3-(Hydroxyphenyl)-propionaldehyden I nach Anspruch 1, dadurch gekennzeichnet, daß man die Umsetzung bei Drücken von 0,1 bis 5 bar durchführt.

4. Verfahren zur Herstellung von 3-(Hydroxyphenyl)-propionaldehyden I nach Anspruch 1, dadurch gekennzeichnet, daß man als basische Katalysatoren Amine verwendet.

5. Verfahren zur Herstellung von 3-(Hydroxyphenyl)-propionaldehyden I nach Anspruch 1, dadurch gekennzeichnet, daß man als basische Katalysatoren sekundäre Amine verwendet.

## Claims

1. A process for preparing 3-(hydroxyphenyl)propionaldehydes of the formula I 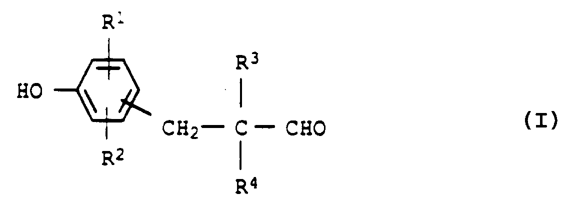 and, where appropriate, for preparing 3-(hydroxyphenyl)propanols of the formula II 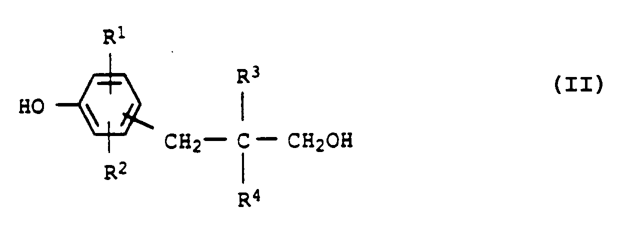 where
R¹, R², R³ and R⁴ are each hydrogen, C₁-C₂₀-alkyl, C₃-C₂₀-cycloalkyl, C₄-C₂₀-alkylcycloalkyl, C₄-C₂₀-cycloalkylalkyl or C₅-C₂₀-alkylcycloalkylalkyl,
R³ and R⁴ are each aryl, C₇-C₂₀-aralkyl, heterocycloalkyl or C₃-C₂₀-heterocycloalkylalkyl, which comprises
a) reacting phenols of the formula III 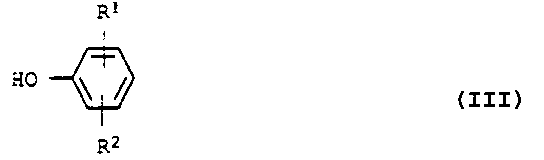 where R¹ and R² have the abovementioned meanings, with 3-hydroxypropionaldehydes of the formula IV 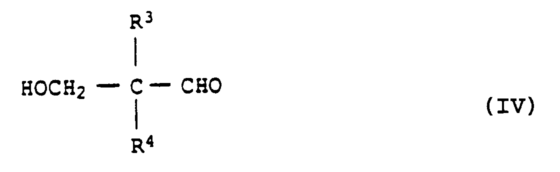 where R³ and R⁴ have the abovementioned meanings, in the presence of a basic catalyst at from 90 to 230°C and under from 0.01 to 50 bar and, where appropriate,
b) treating the resulting 3-(hydroxyphenyl)propionaldehydes of the formula I 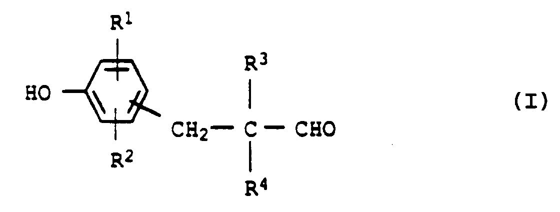 where R¹, R², R³ and R⁴ have the abovementioned meanings, with hydrogen in the presence of a hydrogenation catalyst at from 0 to 250°C and under from 0.1 to 300 bar.

2. A process for preparing 3-(hydroxyphenyl)propionaldehydes I as claimed in claim 1, wherein the reaction is carried out at from 110 to 210°C.

3. A process for preparing 3-(hydroxyphenyl)propionaldehydes I as claimed in claim 1, wherein the reaction is carried out under from 0.1 to 5 bar.

4. A process for preparing 3-(hydroxyphenyl)propionaldehydes I as claimed in claim 1, wherein amines are used as basic catalysts.

5. A process for preparing 3-(hydroxyphenyl)propionaldehydes I as claimed in claim 1, wherein secondary amines are used as basic catalysts.

## Revendications

1. Procédé de préparation de 3-(hydroxyphényl)propionaldéhydes de la formule générale I 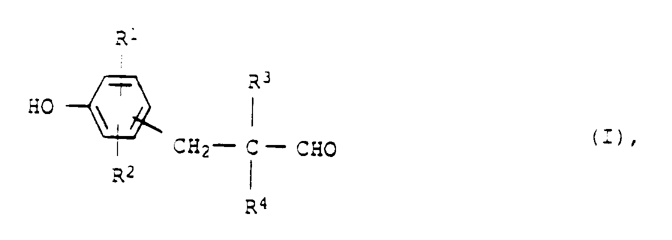 et de préparation éventuelle de 3-(hydroxyphényl)propanols de la formule générale II 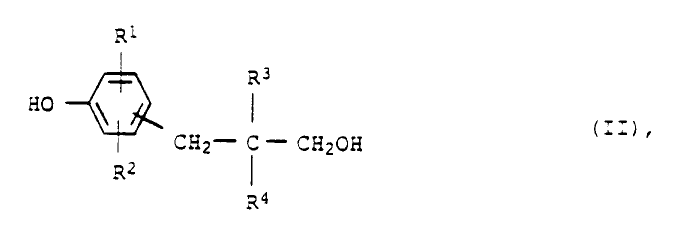 formules dans lesquelles
R¹, R², R³, R⁴ représentent chacun un atome d'hydrogène, un radical alkyle en C₁ à C₂₀, cycloalkyle en C₃ à C₂₀, alkylcycloalkyle en C₄ à C₂₀, cycloalkylalkyle en C₂₀, ou alkylcycloalkylalkyle en C₅ à C₂₀,
R³, R⁴ représentent chacun un radical aryle, aralkyle en C₇ à C₂₀, hétérocycloalkyle ou hétérocycloalkylalkyle en C₃ à C₂₀,
caractérisé en ce que
a) on fait réagir des phénols de la formule III 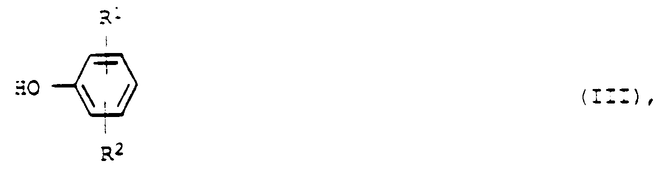 dans laquelle R¹ et R² possèdent les significations qui leur ont été attribuées ci-dessus, avec des 3-hydroxy-proponaldéhydes de la formule générale IV 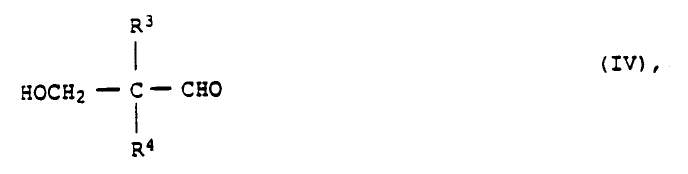 dans laquelle R³ et R⁴ possèdent les significations qui leur ont été attribuées ci-dessus, en présence d'un catalyseur basique, à des températures de 90 à 230°C et sous des pressions de 0,01 à 50 bars et, le cas échéant,
b) on traite les 3-(hydroxyphényl)propionaldéhydes obtenus de la formule générale I 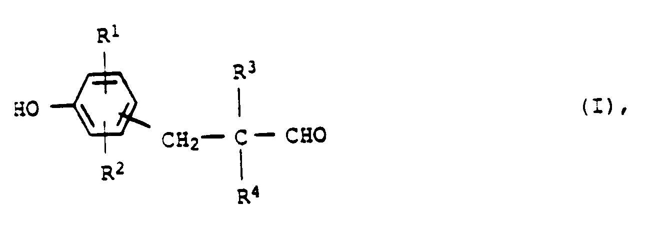 dans laquelle R¹, R², R³ et R⁴ possèdent les significations qui leur ont été précédemment attribuées, en présence d'un catalyseur d'hydrogénation, par de l'hydro
gène à des températures de 0 à 250°C et sous des pressions de 0,1 à 300 bars.

2. Procédé de préparation de 3-(hydroxyphényl)propionaldéhydes I suivant la revendication 1, caractérisé en ce que l'on entreprend la réaction à des températures de 110 à 210°C.

3. Procédé de préparation de 3-(hydroxyphényl)propionaldéhydes I suivant la revendication 1, caractérisé en ce que l'on entreprend la réaction sous des pressions de 0,1 à 5 bars.

4. Procédé de préparation de 3-(hydroxyphényl)propionaldéhydes I suivant la revendication 1, caractérisé en ce que l'on utilise des amines à titre de catalyseurs basiques.

5. Procédé de préparation de 3-(hydroxyphényl)propionaldéhydes I suivant la revendication 1, caractérisé en ce que l'on utilise des amines secondaires à titre de catalyseurs basiques.
